# EUROPEAN PATENT SPECIFICATION

(11) **EP 2 139 455 B1**
(45) Date of publication and mention of the grant of the patent: **19.12.2018**
(21) Application number: 08718773.8
(22) Date of filing: 19.03.2008
(51) Int. Cl.: A61K 9/20, A61K 9/16, A61K 9/50, A61K 31/135, A61K 31/192

(54) **A TABLET HAVING IMPROVED STABILITY WITH AT LEAST TWO ACTIVES**
TABLETTE MIT VERBESSERTER STABILITÄT UND MIT MINDESTENS ZWEI WIRKSTOFFEN
COMPRIMÉ PRÉSENTANT UNE STABILITÉ AMÉLIORÉE AVEC AU MOINS DEUX INGRÉDIENTS ACTIFS

(30) Priority: 24.03.2007 GB 0705714
(43) Date of publication of application: 06.01.2010
(73) Proprietor: Reckitt Benckiser Healthcare (UK) Limited, Slough, Berkshire SL1 3UH (GB)
(72) Inventor: JONES, Huw, Nottingham NG8 2AR (GB); RAJPUT, Gurmeet, Leicester LE3 2GX (GB)
(74) Representative: O'Brien, Niall James
(86) International application number: PCT/GB2008/000933
(87) International publication number: WO 2008/117018

(56) References cited:
- EP-A- 0 459 695
- EP-A1- 0 631 782
- WO-A-01/41733
- US-A1- 2002 177 626

## Description

The present invention relates to a novel dosage form combining two active pharmaceuticals. In particular, a combination of an antihistamine and a NSAID is described.

In many cases, it is perceived to be advantageous to treat a patient with more than one active pharmaceutical in the same dosage form. A single dosage form is more convenient for the patient and has additional safety by reducing the possibility of errors in drug dosage or type caused by complex multi-dosage regimes. However, active pharmaceuticals are often highly chemically active and mixing two or more actives into a single dosage form can present problems in the manufacturing process and/or the stability of the final dosage form.

Each single active has an optimal production process and advantageous excipients which assist shelf life and help maintain efficacy and stability. However, having two or more actives in a single production process may mean compromising the optimal process and conditions for each separate active. This can cause problems with stability, chemical reaction, optimal excipients, etc.

One solution to the problems of interaction is to form a bi-layer tablet by producing each active separately under optimal conditions and then compressing the formed actives and excipients into two separate layers. Such a solution is disclosed in US 2002/177,626, US 4,844,907 and WO 96/24375. Although bi-layer tablets solve some of the problems with multi-active dosage forms they are expensive to produce being effectively two production processes which are merged at the final stage. In addition, they have the drawback of allowing interaction at the bi-layer interface.

A further important aspect of a tablet is the disintegration rate. However, interaction between two actives once the mixed dosage form has been produced can cause the disintegration time to increase and therefore affect the speed of action of the actives.

It is therefore desirable to produce a multi-active dosage form which prevents interaction between the pharmaceutical actives.

According to a first aspect of the present invention there is provided a tablet comprising at least a first and second active, the first active being in the form of a non-aqueous granulate, the second active ingredient being in the form of melt extrudated granules (aqueous or non-aqueous), the said first and second active forms having been blended together, optionally with other excipients, and formed into a tablet wherein the first active is an antihistamine selected from chlorpheniramine, brompheniramine, dexchlorpheniramine, dexbrompheniramine, triprolidine, diphenhydramine, doxylamine, tripelennamine, cyproheptadine, carbinoxamine, bromodiphenhydramine, phenindamine, pyrilamine, and azatadine, and wherein the second active is a suitable non-steroidal anti-inflammatory drug selected from the following categories:
a. the propionic acid derivatives; and
b. the acetic acid derivatives.

According to a second aspect of the present invention there is a provided a method of producing a unit dosage form such as a tablet comprising the steps of:-
(i) producing a non-aqueous granulate of a first active wherein the first active is an antihistamine selected from chlorpheniramine, brompheniramine, dexchlorpheniramine, dexbrompheniramine, triprolidine, diphenhydramine, doxylamine, tripelennamine, cyproheptadine, carbinoxamine, bromodiphenhydramine, phenindamine, pyrilamine, and azatadine;
   producing melt extruded granules of a second active and wherein the second active is a suitable non-steroidal anti-inflammatory drug selected from the following categories:
   a. the propionic acid derivatives; and
   b. the acetic acid derivatives
(ii) blending the said first and second active granules; and
(iii)forming the granules into a unit dosage form.

Preferably, any one or more of steps (i)-(iv) can incorporate optional excipients as defined herein.

Melt extrudate granules are known and described in PCT/EP00/12193 and WO 02/098391. Suitable melt extrusion techniques are described therein. By melt extrudated granules is meant a granular composition obtained by:
(a) melt-extruding said second active, optionally with excipients;
(b) forming a homogeneous extrudate;
(c) cooling said extrudate; and
(d) forming said cooled extrudate into granules.

Preferably, in step (a), the said second active is fully melted. Typically, the extrudate in step (b) is formed into two or more thin ribbons having a depth of 10mm or less and which solidify in 5 minutes or less.

Advantageously, it has been found that blending two actives in the form of a non-aqueous wet granulate and a milled melt extrudate respectively prevents chemical interaction between an otherwise chemically unstable blend. In addition, blending the actives in this manner improves disintegration time between two actives capable of chemically interacting to prevent or hinder the disintegration process.

Preferably, the first active is diphenhydramine or a pharmaceutically acceptable salt thereof, particularly the HCl salt.

The term "NSAID" as used herein is intended to mean any non-narcotic analgesic non-steroidal anti-inflammatory compound, including the pharmaceutically acceptable non-toxic salts thereof, but excluding aspirin, acetaminophen and phenacetin.

While some of these compounds are primarily used at the present time as anti-inflammatory agents and others are primarily used as analgesics, in fact all of the contemplated compounds have both analgesic and anti-inflammatory activity and can be used at appropriate dosage levels for either purpose in the compositions and methods of the present invention. The compounds in groups (1) through (4) typically contain a carboxylic acid function; however, those acids are sometimes administered in the form of their pharmaceutically acceptable acid addition or alkali metal salts, e.g., sodium salts.

The specific compounds falling within the foregoing definition of the non-steroidal anti-inflammatory drugs for use in the present invention are well known to those skilled in the art and reference may be had to various literature reference sources for their chemical structures, pharmacological activities, side effects, normal dosage ranges, etc. See, for example, Physician's Desk Reference, 35th Edition (1981); TheMerck Index, 9th Edition, Merck and Company, Rahway, New Jersey (1976); and Cutting's Handbook of Pharmacology, 6th Edition, Ed.

T.Z. Czacky, M.D., Appleton-Century-Crofts, New York (1979), Chapter 49:538-550.

The propionic acid derivatives for use herein include, but are not limited to, ibuprofen, naproxen, benoxaprofen, flurbiprofen, fenoprofen, fenbufen, ketoprofen, indoprofen,pirpro fen, carprofen, oxaprozin, pranoprofen, miroprofen, tioxaprofen, suprofen, alminoprofen, tiaprofenic acid, fluprofen and bucloxic acid. Structurally related propionic acid derivatives having similar analgesic and anti-inflammatory properties are also intended to be encompassed by this group. Presentlypreferred-members of the propionic acid group include ibuprofen, naproxen, flurbiprofen, fenoprofen, ketoprofen, and fenbufen.

Thus, "propionic acid derivatives" as defined herein are non-narcotic analgesics/nonsteroidal anti-inflammatory drugs having a free -CH(CH3)COOH or -CH2CH2COOH group (which optionally can be in the form of a pharmaceutically acceptable salt group, e.g., -CH(CH3)COO-Na+ or-CH2CH2COO-Na+), typically attached directly or via a carbonyl function to a ring system, preferably to an aromatic ring system.

The acetic acid derivatives for use herein include, but are not limited to, indomethacin, sulindac, tolmetin, zomepirac, diclofenac, fenclofenac, alclofenac, ibufenac, isoxepac, furofenac, tiopinac, zidometacin, acemetacin, fentiazac, clidanac and oxpinac. Structurally related acetic acid derivatives having similar analgesic and anti-inflammatory properties are also intended to be encompassed by this group. Presently preferred members of the acetic acid group include tolmetin sodium, sulindac and indomethacin.

Thus, "acetic acid derivatives" as defined herein are non-narcotic analgesics/non-steroidal anti-inflammatory drugs having a free -CH2COOH group (which optionally can be in the form of a pharmaceutically acceptable salt group, e.g., -CH2COO-Na+), typically attached directly to a ring system, preferably to an aromatic or heteroaromatic ring system.

Of course, it will be appreciated by those skilled in the art, that any of the foregoing compounds may be utilized in the form of pharmaceutically acceptable salts thereof.

More preferably, a NSAID is used such as a phenyl propionic acid selected from ibuprofen, naproxen, ketoprofen and flurbiprofen, most preferably, ibuprofen is used.

It may be found that the crystalline form of the second active changes on melting and then cooling. For example, the crystals become smaller and the surface area of the second active in the melt granule is increased compared to that of conventional crystals of the second active. In addition, on cooling, the changes to the crystalline structure lead to a more porous granule.

The crystalline structure of the melt granules formed from solidifying fully melted second active differs from the crystalline structure where the second active is only partially melted. In the case of partial melting, the crystalline structure of the melted second active is interrupted by the non-melted second active, thus providing that the second active does not have a single crystalline structure. The second active is fully melted so that on cooling, a single continuous phase of the second active is formed. That is to say the crystalline structure of the second active is not interrupted by another crystalline second active structure. The melt granules are thus in the form of a solidified melt of second active, comprising said second active solely as a continuous phase.

The invention allows the formulation of any relatively low melting second active into an acceptably tasting, readily disintegrating composition. It is generally envisaged that the melting point of such compounds will be low enough to allow the melting thereof using standard equipment. It is also important that there is not a deleterious effect on the second active itself or any ingredients incorporated in the molten second active, for example a disintegrant. Thus, typical melting points of the low melting second active would be expected to fall within the range 30-300°C.

Preferred second actives have lower melting points so that the melting process does not use significant amounts of energy, which thus reduces production costs. A favoured class of compounds are the 2-arylpropionic acids which are generally substantially insoluble and have poor taste properties. Preferred second actives have melting points in the range 30-200°C (such as racemic naproxen, melting point 156°C), more preferably 30-150°C, further preferably 40-120°C (such as racemic flurbiprofen, melting point 114°C), most preferably 40-100°C (such as racemic ibuprofen (melting point 75-77°C), S(+)-ibuprofen (melting point 52-54°C) and racemic ketoprofen (melting point 96°C)). Preferred low-melting second actives are naproxen, ketoprofen, flurbiprofen, ibuprofen. Preferably the second active is in the form of a racemic mixture or an enantiomer (especially the S(+)-enantiomers) thereof. Salts of racemic second actives and enantiomers thereof may also be used. Preferred salts are the sodium salts, potassium salts and the lysine salts.

The invention is especially adapted for an ibuprofen medicament. The term "ibuprofen medicament" preferably includes racemic ibuprofen and S(+)-ibuprofen and their sodium, potassium and lysine salts which have low melting points and a very poor after-taste in the mouth and throat. Most advantageous results are obtained with racemic ibuprofen which has a high dosage combined with poor solubility properties.

In step (a) the second active is melted and extruded. Under conditions of pressure, the drug may be melted at a temperature below its normal melting point. The maximum temperature is determined by the stability of the molten drug and ingredients combined therewith. The drug may be heated to any convenient temperature. Generally, the higher the temperature, the more quickly the drug will melt although this must be balanced by the energy input required to heat the drug. For highest efficiency, it is generally envisaged that the second active will be heated to not more than 50°C, preferably 1-25ºC and more preferably 5-20°C, above its melting point to keep energy costs to a minimum. A preferred heating range is 30-180ºC, more preferably 35-140ºC and further preferably 40-120ºC.

The second active is generally melted in a heated extruder barrel having an inlet for the solid drug and an outlet for the molten extrudate. The barrel may be divided into different heating zones as desired. In addition, the work on the second active by the screw configuration in the extruder will also contribute to melting the second active, thereby reducing its external applied temperature requirement. Accordingly the extruder barrel may be heated to a temperature less than the melting point of the SECOND ACTIVE. For example, the normal melting point of racemic ibuprofen is 75-77°C, however under conditions of force/pressure (such as may be encountered in an extruder or similar processing device), the external applied heat necessary to melt the ibuprofen may be reduced significantly through the mechanical heat generated by the intense mixing action within the extruder. It is generally envisaged that the extruder will be heated to a temperature not less than 25°C below the melting point of the drug, preferably in the range from 20°C below the melting point of the drug to 50°C above the melting point of the drug, more preferably from 15ºC below the melting point of the drug to 25ºC above its melting point and most preferably to a temperature in the range of 10°C on each side of the melting point of the drug. Some extruders allow different zones to be heated to different temperatures in the extruder. These temperatures can be chosen as desired to ensure that the second active is fully melted in step (a). Preferably, the drug and optional excipients, for example a disintegrant, are heated to a temperature in the range 80-130°C, more preferably 100-120°C to melt said drug. When the second active is ibuprofen it may conveniently be heated in the range 50-130°C, more preferably 60-100°C. The temperature of the ibuprofen in the extruder barrel is preferably in the range 66-96°C, preferably 70-82ºC.

The extruder may also have one or more cooling zones. The cooling zones may be necessary to remove the heat generated by the kneading action on the material being extruded, particularly to ensure that there is a good flow of material into the extruder and out from the extruder.

In a preferred process according to the present invention, the extruder is provided with a cooling zone and a heating zone. Further preferably, there is provided a cooling zone at the inlet portion of the extruder so that the material entering the extruder may be conveyed or transferred along the extruder to a heated zone. In the cooling zone, the internal heat generated within the material being extruded is carried away so that partial melting of the second active cannot occur which may be detrimental to the throughput of material in the extruder. Preferably, the extruder is provided with a cooled transfer zone and a heated melting zone.

In a further preferred process, there is provided a heated zone at an end portion of the extruder at or adjacent the outlet. The extruded material may be heated to ensure that the extrudate passing through the extruder outlet is sufficiently heated so that the temperature difference between the molten extrudate and extrudate cooling means is maximised as appropriate to optimise the cooling process. For example, the barrel may be heated to cause the extrudate passing through the outlet to be preferably fully molten or substantially fully molten. The pressure within the extruder may cause a lowering of the melting point of the second active. Accordingly, preferably, the temperature of the extrudate passing through the outlet is in the range of 20ºC on each side of the normal melting point of the drug, preferably within 10ºC on each side of the melting point of the drug.

The extruder is suitably provided with at least one screw shaft provided with means arranged to generate heat within the second active. This may usually be achieved by a combination of kneading paddles and helical screws. Generally, it is preferred to provide helical screws at the inlet portion to convey the material away from the inlet. The material may be extruded in the extruder barrel with screws and/or with paddles. It is preferred to use more than one screw shaft, for example a twin-screw shaft, to maximise the extrusion effect on the material being extruded. The use of paddles also maximises the shear effect on the material being extruded. The paddles may be offset at any desired angle or combination of angles to generate internal heat within the drug as appropriate to melt the drug. The configuration and/or size of the paddles will depend on factors such as the diameter and/or length of the extruder, the ratio of the length to the diameter, the extruder speed, the torque applied and the desired temperature to melt the second active. The screws and/or the paddles may be in the forward and/or reverse direction to maximise the pressure within the mixing zone as desired.

A preferred arrangement comprises helical transfer screws at inlet portion of the extruder, a plurality of paddles which may have differing sizes and degrees to which they are offset and further helical transfer screws at the outlet portion to convey the extrudate out of the extruder. Further preferably the helical transfer screws at the outlet portion may comprise a reverse helix followed by a forward helix.

A preferred feature of step (a) is that a disintegrant is also combined with said drug in molten form. In a further preferred feature of step (a), at least one of a surfactant and a diluent is combined with said drug in molten form.

Conveniently, the second active may be de-aerated as it is transferred to the melting zone.

In a further process, the second active may be combined with optional excipients, eg a diluent, and then heated together until said second active is molten. In yet a further process the second active and optional excipients may be combined in the solid state and extruded together until said second active is molten and any further desired excipients uniformly blended with the mixture.

Alternatively, the second active may be melted before it is combined with any optional excipients.

In step (b), a homogeneous extrudate is formed which passes out through the outlet of the extruder. Preferably, the second active is fully molten as it exits the extruder. The extrudate may consist of said second active, without additional ingredients, wherein the second active is present as a single continuous phase. Optionally, the extrudate may contain additional excipients, for example one or more of a disintegrant, a surfactant and a diluent, which are blended within the molten second active.

In step (b), the extrudate is formed into two or more thin ribbons. This is preferably achieved by passing the molten extrudate through channels at the outlet which form streams or ribbons of extrudate which may be directed onto the cooling means, preferably a cooling belt or a cooling drum.

The ribbons of molten extrudate are cooled rapidly by said cooling means, ie the ribbons solidify in 5 minutes or less, preferably in 3 minutes or less, more preferably in 1 minute or less (eg 0-60 seconds), preferably in 50 seconds or less (eg 1-50 seconds), more preferably 1-40 seconds and most preferably 1-30 seconds.

Suitably, the width of each ribbon of molten extrudate is greater than the depth of the ribbon so that cooling is optimised. The width of each ribbon will depend, at least to some extent, on the viscosity of the molten extrudate. Preferably, each ribbon of molten extrudate has a depth on the cooling means of 10 mm or less, preferably up to 6 mm (for example 0.1-6 mm), preferably 0.5-5 mm, for example 3-4 mm and most preferably 1-3 mm, for example 2 mm.

Cooling will normally occur first on the side of the ribbon proximate the cooling means. Accordingly, usually the lower surface of the ribbons solidifies while the upper surface of the ribbon is still molten. As the ribbon is further cooled, the extrudate solidifies throughout its depth.

To maximise output, a plurality of ribbons are provided extending parallel to each other, for example on a cooling belt. Preferably, there are more than two ribbons, for example three, four, five, six, seven, eight, nine or ten or more ribbons according to the size of the extruder. The number of ribbons may be limited by the width of the ribbon formed and the whole width of the cooling means which provides for a maximum number of ribbons. It has been found that the ribbons of molten second active do not spread on the cooling means, accordingly there requires only a small space between the ribbons.

As hereinabove discussed, it is preferred to have a significant temperature difference between the molten extrudate and the cooling means as the extrudate comes into contact with the cooling means, for example at least 25ºC, preferably at least 35ºC, more preferably at least 45ºC and most preferably at least 55ºC. The upper end of the above ranges is limited by the melting point of the drug, but it is not desired to heat the extruded material to too high a temperature as the extra energy costs will not be balanced by any processing advantage. Accordingly, a practical upper limit to each of the above ranges is 100ºC, more usually 80ºC.

Generally, it is expected that the molten mixture will be cooled to a temperature below the melting point of the drug before being formed into granules. The molten second active may be poured onto cooling trays which may be static or continuously moving. Static trays may be placed in cooling cabinets. Moving trays or belts may have additional cooling means, such as cooled water. The cooled melt forms a solid and may be scraped off the belt or collected as it falls off one end of a continuously moving belt. Preferably the molten drug mixture may be cooled by passing the molten mixture onto a moving cooling belt, preferably a continuously rotating cooling belt. Preferably, the belt is cooled by water. The water may be applied to the underside of the belt along its length or partially along its length as desired and according to the length of the belt, the quantity of molten drug mixture and the speed of the belt. It is especially preferred to cool the molten drug mixture at least initially by cooling means, for example until it has started to solidify. Advantageously, the belt is water-cooled along substantially the whole of its length and it is of minimum length required (e.g. 3-7m) to allow it to cool to the solid state.

The solidified melt may be formed into granules by a plurality of methods. For example, it may be pulverised or comminuted into granules. It may be milled and/or sieved. If it is cooled on a moving belt or drum, the cooled melt may be broken into conveniently sized pieces, followed by milling and or sieving.

The granular composition may be sieved to ensure that the melt granules are of the appropriate size for efficient tabletting. The granules produced on cooling the molten drug are preferably of a suitable size for tabletting, preferably in a standard large scale tabletting machine. The melt granules in the granular composition preferably have a mean particle size in the range 10-2000µm, more preferably 50-1000µm and most preferably 100-400µm. Valuable results are achieved when the bulk density of the melt granules is in the range 0.1-1gml⁻¹, more preferably 0.3-0.6gml⁻¹. Further preferred properties are obtained when the tapped density is in the range 0.3-0.7gml⁻¹ (more preferably 0.4-0.6 gml⁻¹). Further, preferably the melt granules have a porosity of 0.5-2.0 g/ml.

The proportion of second active in the melt granules will depend on the dose desired for therapeutic effect. Low dose drugs, such as flurbiprofen and ketoprofen may form as little as 1% by weight if a relatively large dosage form is required. However, particular advantages of the present invention are obtained by allowing a reduction in the number and/or amount of excipients. Accordingly the second active may form up to 100% w/w of the melt granules. Accordingly, it is generally envisaged that the second active will generally form 10-100% w/w of the melt granule, preferably 50-100% w/w, more preferably 70-100% of the melt granule. A preferred feature of the invention is that low-melting, high dose second actives, such as ibuprofen, can be formulated with a disintegrant into smaller dosage forms. Accordingly, the second active will suitably form 60-95% w/w of the melt granules, preferably 70-95% w/w and further preferably 80-95% w/w of the melt granules.

The solidified melt granules may be formulated directly into the combined formulation or they may be combined with an extra-granular composition and formulated into a unit dose. The melt granules are preferably combined thoroughly with an extra-granular composition containing the first active so as to form a uniform mixture of ingredients. This may be achieved by conventional mixing and blending techniques. Examples of apparatus that may be used to facilitate this process are: Ribbon Blender, IBC Blender, V-Bender and Plough Benders. Examples include filling of the loose powder mixture into a sachet or a capsule or compressing it into a tablet. Tablets are the preferred unit dosage form according to the invention. They may be swallowed or they may be chewed. It has unexpectedly been found that the taste of the second active has been substantially masked which allows the dosage form to be maintained in the oral cavity for a period of time whilst the formulation is swallowed.

The compressed tablet composition of the present invention may optionally be coated with a film coat, for example based on a conventional cellulose polymer such as hydroxypropylmethylcellulose, or a conventional sugar coat, for example based on sucrose or lactose.

The granular composition of the invention may also comprise a disintegrant. The addition of a disintegrant further improves the rate at which the tablet breaks up when added to a liquid medium. Examples of disintegrants include one or more of starch and modified starch (such as wheat starch, maize starch, potato starch), sodium starch glycolate, low-substituted hydroxypropyl cellulose, alginic acid, cross-linked polyvinylpyrrolidone, magnesium aluminium silicate, bentonite and croscarmellose sodium. Preferred disintegrants are those which swell on the action of water, thus causing the ingredients in the tablet to be pushed apart and out into the aqueous disintegration medium. Preferred examples of disintegrants are croscarmellose sodium and/or sodium starch glycolate, especially croscarmellose sodium. The disintegrant is present at an effective disintegrating amount, for example up to 50% w/w of the formulation (eg 1-50% w/w), more preferably 1-25% w/w, further preferably 2-20% w/w and most preferably 2-15% w/w of the formulation.

The disintegrant is an optional ingredient of the melt granules and/or the extra-granular composition. The disintegrant will suitably form 0.1-25% w/w of the melt granules, preferably 3-15% w/w and most preferably 4-10% w/w of the melt granules. The disintegrant may be present in the extra-granular composition in an amount of 0.1 to 25% w/w, preferably 1-15%, more preferably 2-10% w/w.

The ratio of first and second active to disintegrant will depend on the proportion of the first and second active in the dosage form. Thus, depending on the dosage of the two actives, it can be expected to fall in the range 20:1 to 1:20, conveniently 10:1 to 1:10. For relatively high dose drugs, the ratio of first and second active to disintegrant may be in the range 20:1 to 2:1, preferably 10:1 to 5:1 parts by weight. For relatively low dose drugs, the ratio of first and second active to disintegrant is suitably 1:10 to 10:1, preferably 1:1 to 1:5 parts by weight.

Although not necessary for the production of compositions according to the present invention, if desired, the dosage form may further comprise a diluent. The diluent, may be water-soluble or water-insoluble. Suitable water-soluble diluent materials include the sugar alcohols (such as xylitol, sorbitol, mannitol, erythritol), sugars (such as sucrose, fructose, lactose, dextrose), cyclodextrin, maltodextrin and salts of organic acids (eg sodium citrate and potassium citrate). Lactose, sodium citrate and potassium citrate are particularly preferred water-soluble diluents. Suitable water-insoluble diluent materials include cellulose derivatives (such as microcrystalline cellulose) starch and derivatives thereof (such as pre-gelatinised starch), dicalcium phosphate, tricalcium phosphate, calcium sulphate, calcium carbonate. Microcrystalline cellulose and dicalcium phosphate are preferred water insoluble diluents.

The diluent may preferably include a basic ingredient such as an alkali metal salt for example an alkali metal carbonate, bicarbonate or citrate, present to an extent of up to 50% by weight (eg in the range 1-50% by weight), preferably up to 40% by weight (eg in the range 1-40% by weight) of the formulation (more preferably 2-35% w/w and most preferably 10-20% w/w). Preferably, the alkali metal salt is sodium or potassium. Further preferably, the salt is a citrate, carbonate or bicarbonate salt of sodium or potassium, more preferably sodium bicarbonate or citrate. The ratio of first and second active (especially diphenhydramine/ibuprofen medicament) to alkali metal salt may be in the range 100:1 to 1:1 parts by weight, preferably 5:1 to 1:1 parts by weight. Preferably, the alkali metal salt is incorporated in any amount up to an equimolar amount with respect to the first and second active (eg ibuprofen). Conveniently, a sub-molar amount of alkali metal salt is incorporated. Thus the alkali metal compound may form up to 100% w/w of the first and second active, preferably 50% w/w, more preferably up to 10% w/w, of the first and second active. In a preferred compressed tablet according to the present invention, the first and second active (especially a diphenhydramine/ibuprofen medicament) is in admixture with the alkali metal salt. The alkali metal salt is preferably incorporated in the melt granules.

In a formulation adapted to disperse in water prior to administration, the level of diluent may be quite high, for example up to 50% (such as 0-50% w/w, preferably 0-40% w/w) by weight of the formulation in order to achieve the desired dispersing properties. Preferably, the diluent does not form greater than 25% by weight of the formulation (eg 0-25% w/w), as it adds to the costs of the composition and to production costs. Thus, to minimise costs it may be preferred that the diluent is added to the formulation in an amount of 0-20% by weight of the formulation, more preferably 0-10% w/w. If present, it may be preferably used to an extent of 0.1-25% by weight of the formulation, more preferably 0.1-20% w/w, further preferably 0.1-10% w/w and most preferably 1-5% by weight of the formulation.

The diluent may be contained in the non-aqueous granulate, the melt granule, and/or may be part of the extra-granular composition or may be incorporated as desired in all components. If desired, the diluent may preferably be added in an amount up to 30% w/w of the extra-granular component (ie 0.1-30% w/w, although to minimise the size and cost of the dosage form, it is desired to include a minimum amount of such additional excipients. Accordingly, if employed, the diluent may suitably be included in the extra-granular composition in the range up to 30% w/w (ie 0.1-30%), preferably 0.1-15% w/w, more preferably 0.1-10% w/w. As discussed hereinabove, the diluent may be present in the non-aqueous granulate and/or melt granules, for example in an amount of 0-30% w/w (such as 0.1-30%) by weight of the non-aqueous granules and/or melt granules. If present, the diluent conveniently forms 1-20%, more preferably 1-10% w/w of the non-aqueous granulate and/or melt granules.

The formulation may also include a surfactant, in the amount appropriate to the properties of the surfactant, preferably 0.05-10% by weight of the formulation. The surfactant may be included in the non aqueous granulate, melt granules and/or the extra-granular composition. Preferred surfactants are sodium lauryl sulphate, poloxamer, hydrogenated castor oil and derivatives thereof, polyoxyethylene surfactants (including polyoxyethylene oils, fatty acid esters, including stearates) and sorbitan esters. They may be used to an extent of 0.05-5% w/w, preferably 0.1-3% w/w, more preferably 0.2-2% w/w of either one or more or all of the melt granules, non-aqueous melt granulate and the extra-granular composition.

The extra-granular composition comprises the ingredients incorporated in the formulation which are not contained in the solidified melt granules and/or non-aqueous granulate. The ingredients of the extra-granular composition may be mixed with the active granules simultaneously or at sequential stages in the process to prepare the formulation. A particular advantage of the present invention is preferably that all the ingredients of the extra-granular composition are combined with the active granules at a single stage in the manufacturing process. Also, it is preferred that at this stage, the ingredients in the extra-granular composition are combined sequentially with the active granules. The formulation comprises a uniform mixture of active granules and extra-granular composition. The extra-granular composition is suitably distributed evenly throughout the formulation.

The extra-granular composition may also comprise flow acids such as colloidal silicon dioxide and talc. The colloidal silicon dioxide is insoluble and suitably has a surface area greater than 50 m²g⁻¹, more preferably greater than 100 m²g⁻¹', especially in the range 150-250 m²g⁻¹.

Optionally a lubricant may be incorporated in the extra-granular composition for mixing with the melt granules. Conventional lubricants for first and second actives may be used for example stearic acid, sodium lauryl sulphate, polyethylene glycol, hydrogenated vegetable oil, sodium stearyl fumarate, magnesium stearate or calcium stearate. These may be present in an amount from 0.05-5% by weight, preferably 0.1-2% by weight of the formulation. Anti-adherents such as talc, may further be included in an amount of up to 4% by weight of the dosage form, for example 0.5-2% by weight of the dosage form, preferably as part of the extra-granular component.

Other conventional tabletting excipients known to the person skilled in the art may be incorporated in the compressed tablet composition according to the present invention as desired, although it will be appreciated that a prime advantage of the present invention is that the number of excipients necessary to achieve a quickly disintegrating tablet with good dissolution characteristics is minimal.

A disintegrant is preferably the sole ingredient incorporated within the first and second active (preferably diphenhydramine/ibuprofen) granules or they may be combined with a diluent and optionally a surfactant and other tabletting excipients. Accordingly, in one preferred embodiment, the granules may comprise (be made up of) greater than 90% w/w of the first and second active and disintegrant (ie 90-100% w/w). Preferred melt granules comprise a second active (preferably ibuprofen), a disintegrant and optionally a surfactant and/or a diluent. In a further preferred embodiment, the formulation comprises greater than 90% w/w (ie 95-100% w/w) of the combination of first and second active, acidic component and disintegrant. Further preferably, the formulation consists essentially of (i.e. 98-100% w/w) of the combination of first and second active and disintegrant. Further preferred melt granules consist essentially of a second active (preferably ibuprofen), a disintegrant and a surfactant. Further preferred granules consist essentially of a second active (preferably ibuprofen), a disintegrant and a surfactant. Preferably the second active is ibuprofen.

Ibuprofen and its derivatives are primarily anti-inflammatory, analgesic and anti-pyretic agents but have also been proposed for other therapeutic uses, including the treatment of periodontal bone loss, pruritus and Alzheimer's disease. The dosage forms of the present invention are therefore indicated for use in the treatment of all therapeutic uses for which ibuprofen is effective, including rheumatoid arthritis, osteoarthritis, ankylosing spondylitis, seronegative arthropathies, periarticular disorders and soft tissue injuries. They may also be used in the treatment of postoperative pain, postpartum pain, dental pain, dysmenorrhoea, headache, migraine, rheumatic pain, muscular pain, backache, neuralgia and/or musculoskeletal pain or the pain or discomfort associated with the following: respiratory infections, colds or influenza, gout or morning stiffness.

Accordingly, in another aspect of the present invention there is provided a composition according to the present invention for use in the treatment of pain and/or inflammation and/or fever. Furthermore, the invention also provides a method of treating pain and/or inflammation and/or fever comprising the administration of a composition according to the present invention to a mammal in need thereof.

Unit dosages for effective therapy are known to those skilled in the art for each second active. For example, they may comprise the second active to an extent of 5mg, 10mg, 12.5mg, 25mg, 50mg, 100mg, 150mg, 200mg, 250mg, 300mg, 350mg, 400mg, 500mg, 600mg and 800mg. Where derivatives are employed, normally the precise unit dosages are chosen to give the equivalent second active doses given above. For the treatments described herein the maximum daily dose of ibuprofen is generally 3200 mg. A single unit daily dose may be 100 mg. Preferred unit doses are in the range 100-400 mg, more preferably 100-300 mg and especially 200 mg ibuprofen. The maximum daily dose of flurbiprofen is generally 300 mg. A single unit dose may be 12.5 mg. Preferred unit doses are in the range 12.5-150 mg, more preferably 25-100 mg and especially 50 mg flurbiprofen. The maximum daily dose of naproxen is generally 1500 mg. A single unit daily dose may be 125 mg. Preferred unit doses are in the range 220-750 mg, more preferably 220-500 mg and especially 220-250 mg naproxen. The maximum daily dose of ketoprofen is generally 200 mg. A single unit dose may be 25 mg. Preferred unit doses are in the range 25-100 mg, more preferably 25-75 mg and especially 50 mg ketoprofen.

In a preferred feature, the extra-granular composition further comprises at least one of silicon dioxide, stearic acid or a salt thereof, a surfactant and diluent.

In a preferred process according to the present invention, said second active comprises ibuprofen.

Preferably, the non-aqueous wet granulate includes, apart from the first active, other necessary excipients such as disintegrants selected from alginic acid, calcium phosphate- tribasic, carboxymethylcellulose calcium, carboxymethylcellulose sodium, cellulose powdered, chitosan, colloidal silicon dioxide, croscarmellulose sodium, croscarmellose sodium, crospovidone, docusate sodium, guar gum, hydroxypropyl cellulose, magnesium aluminium silicate, methylcellulose, microcrystalline cellulose, povidone, sodium alginate, sodium starch glycolate, starch - pregelatinized; binders selected from lactose BP, acacia, alginic acid, carbomers, carboxymethylcellulose sodium, carageenan, cellulose acetate phthalate, ceratonia, chitosan, confectioner's sugar, cotton seed oil, dextrates, dextrin, dextrose, ethylcellulose, gelatin, glucose liquid, glyceryl behenate, guar gum, hydroxyethylcellulose, hydroxyethylmethyl cellulose, hydroxypropyl cellulose, hypromellose, Mg aluminium silicate, maltodextrin, maltose, methylcellulose, microcrystalline cellulose, poloxamer, polydextrose, polyethylene oxide, polymethacrylates, povidone, sodium alginate, starch - pregelatinized, stearic acid, sucrose, sunflower oil, zein.

Suitable solvents for the non-aqueous granulate include:-isopropylalcohol (IPA), and other solvents known to those skilled in the art of granulation or mixtures thereof.

Preferably, isopropyl alcohol is used as the wet granulation solvent.

By non-aqueous is meant that the wet granulation took place in a solvent having less than 30% water, more preferably, less than 10% water, most preferably, less than 1% water.

Preferably, the melt extrudate may be produced by any suitable technique which extrudes molten active, optionally, together with other excipients wherein the said other excipients may be extruded whilst molten or non-molten and are preferably, non-molten. By melt granules is meant a solid melt extrudate which has been reduced to a suitable particle size using any suitable process, preferably, by physical breakdown such as grinding, bombardment, etc.

Preferably the dosage range for the first active in the table may be any suitable dosage in accordance with safety and efficacy. Preferably, the dosage range in the tablet for the second active may be any suitable dosage in accordance with safety and efficacy. Suitable ranges for both actives independently include 0.1mg-2000mg, more preferably, 0.5-1000mg, most preferably 1mg-500mg.

A preferred dosage range for ibuprofen in a single tablet is 25-500mg, more preferably, 50-400mg, most preferably, 200-300mg, typically 250mg.

A preferred dosage range for diphenhydramine in a single tablet is 1-200mg, more preferably 5-100mg, most preferably 20-50mg, typically 50mg. Preferred daily dosage of diphenhydramine is 50-400mg, more preferably 100-300mg, especially 200mg.

The ratio of first and second active in a single tablet can be within a broad range depending on safety and efficacy, for instance, the ratio of first:second active can be between 1:1000 and 1000:1 wt/wt, more preferably 1:100 and 100:1, most preferably 1:50 and 50:1.

In an ibuprofen:diphenhydramine combination the preferred ratio is 50:1 to 1:2, more preferably 20:1 to 1:1, most preferably 15:1 to 2:1, typically 8:1.

Preferably, the ratio of wet granulate:melt granules is in the range 1:10 to 5:1, more preferably, 1:5 to 2:1, most preferably 1:1 to 1:3.

Preferably, optional other excipients may be selected from those known to the person skilled in the art.

Typically, other excipients include lubricants such as stearic acid and calcium stearate.

The invention will now be illustrated without limitation, with reference to the accompanying examples and drawings in which:-
Figure 1 is a schematic side elevation of melt extrudate production apparatus;
Fig 2 represents a perspective view of an extruder with the top portion of the barrel removed showing details of the screw configuration.
Fig 3 represents a plan view of an end plate mounted on the extruder.
Fig 4 represents a perspective-view showing the extrudate being fed from the endplate mounted on the extruder onto the cooling belt and using ribbons in the extrudate.

### Disintegration Time (Minutes)

The disintegration time is measured using the disintegration method described in the European Pharmacopoeia 1986, Ref V.5.1.1 (updated 1995) using tap water (pH approximately 7) as the liquid. The method measures the time (seconds) by which six tablets prepared with each Example formulation disintegrates.

### Extrusion apparatus

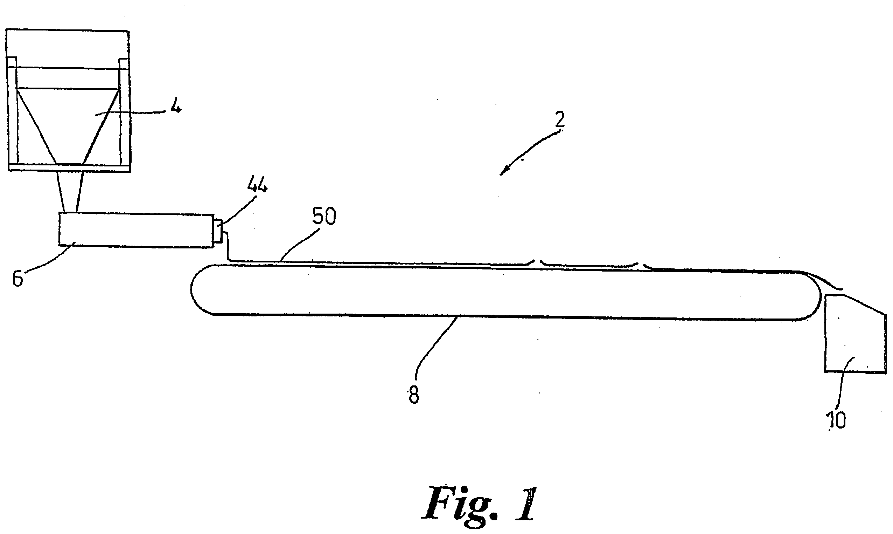

Referring to Figure 1, the extrusion apparatus (2) comprises a feeder hopper (4), an extruder (6) with an endplate (44) mounted thereon, a cooling belt (8) to cool the extrudate (50) and a collection hopper (10).

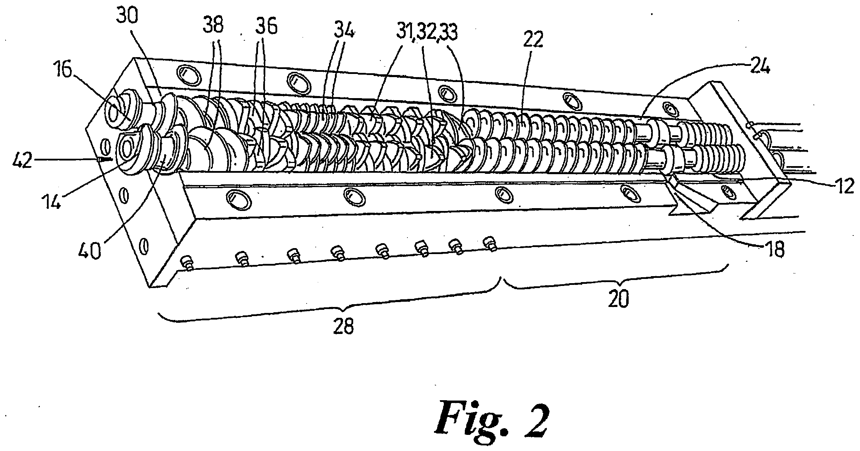

Referring to Figure 2, the extruder was an APV MPC40 twin-screw extruder (having a 40mm diameter barrel; a length: barrel diameter ratio of 1:20 and used at an extruder speed of 600 rpm). The extruder included an extruder barrel (12), twin-screw shafts (14,16), a powder inlet (18), a transfer zone (20) (44cm in length) enclosed by a water cooled jacket (24), a heated mixing zone (28) (36cm in length) enclosed by a thermal jacket (30) and an extruder outlet (42).

In the transfer zone (20), the screw-shafts are each provided with intermeshing forward rotating helical screws (22) by which the second active, optionally mixed with excipients, is transferred to the heated mixing zone (28). During the transfer, the second active is de-aerated and the heat generated within the active is removed by the water-cooled jacket (24). In the heated mixing zone (28), the screw-shafts are each provided with an arrangement of positive scraping paddles (31,32,33) offset at 30°,60° and 90° respectively, to knead material in the heated mixing zone, half size paddles (34) to further work the mixture and to generate internal heat within the drug mixture, intermeshing kneading paddles (36) arranged in such a way as to provide a thorough mixing action, reverse helical screws (38) to provide a reverse flow to maintain pressure within the mixing zone and further helical forward rotating screws (40) to carry the molten extrudate through the outlet (42) of the extruder .

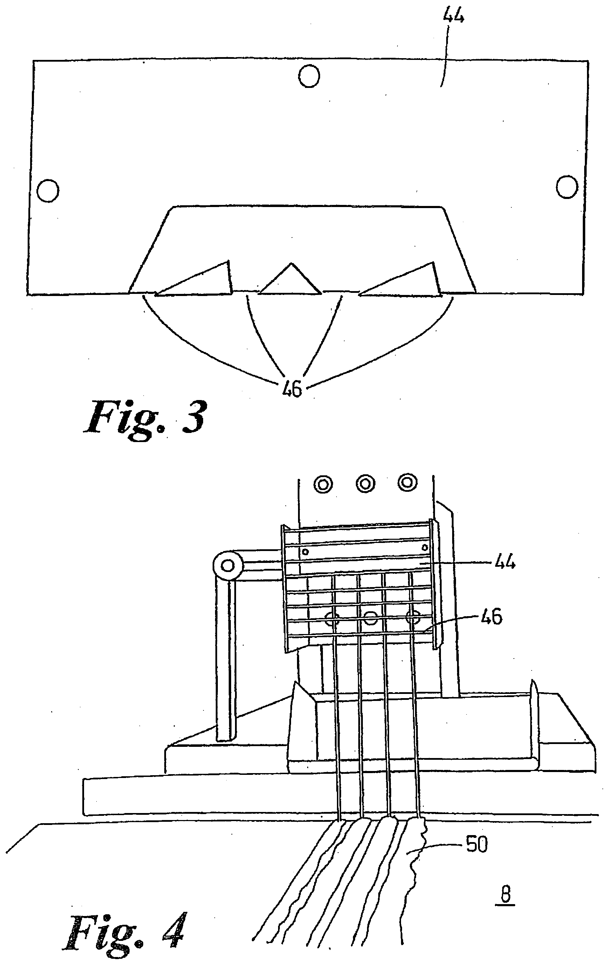

Referring to 3, a more specific embodiment using ribbon extrudates is shown. The endplate (44) is mounted at the end of the extruder barrel so that the liquid extrudate passes into the endplate from the extruder barrel. The endplate is heated by thermal transfer from the main extruder block. Channels (46) in the endplate divide the molten extrudate into a plurality of thin streams. Referring to Figure 4, the streams of molten extrudate flow from the endplate (44) to form ribbons (50) on a continuously rotating stainless steel cooling belt (8) (at a rate of 6.6m/min) water-cooled along its entire length. The length of the belt from the endplate (44) to the collection hopper (10) is 4m. A protection grill (46) protects the interruption of the extrudate flowing out of the endplate.

### Comparative Example 1

### Ibuprofen 200mg Diphenhydramine HCl 25 mg tablets

Comparative Example 1 is chemically stable but physically unstable. Table 1 shows the composition of comparative Example 1. Table 3 shows the level of Benzophenone (Degradation product of diphenhydramine HCl) and the disintegration time of the tablets in comparison with Nytol tablets (comparative 3), after 6 months at 4°C and 40°C/75%RH.

**Table 1. Comparative Example 1**

| Ingredient | mg/tab | % w/w |
|---|---|---|
| Diphenhydramine HCL | 25 | 8.27 |
| Ibuprofen 38 | 200 | 66.23 |
| Croscarmellose sodium | 30 | 9.93 |
| Sodium Citrate BP Pdr | 43.5 | 14.40 |
| Sodium lauryl Sulphate | 0.5 | 0.17 |
| Colloidal silica anhydrous | 1 | 0.33 |
| Stearic Acid | 2 | 0.66 |
| Total | 302 mg | |

### Method of Manufacture

Ibuprofen, croscarmellose sodium, sodium citrate and sodium lauryl sulphate were thoroughly pre-mixed in a tumble blender to form a homogeneous mixture. The solid powdered mixture was fed through the feeder hopper (4) into the powder inlet (18) at a rate of 120-180 kg/hr into the water-cooled transfer zone (20) of the extruder. In the transfer zone (20), the powdered mixture was thoroughly kneaded between the helical screws and transported to the heated mixing zone of the extruder where the extruder barrel was heated to 100 °C. In the heated mixing zone, the mixture ,was worked between the intermeshing paddles (31, 32, 33) which generated shear-induced heat to ensure that the ibuprofen was fully molten. The temperature of the extruded material reached approximately 10-30°C. The extruded material was then further worked by the half-size paddles (34), the kneading paddles (36) and the reverse helical screws (38) which created a back pressure to the system, further pressurising the heated zone. The extrudate was fed by the forward rotating screws (40) through the outlet of the extruder (42) under pressure and through an endplate.

The lengths of solidified extrudate were milled & sieved through a screen with a round hole size of 1 mm to provide a granulate material.

The powdered Diphenhydramine HCl is then triturated in to the extrudate. Stearic acid and colloidal silica anhydrous are added and the whole mixture blended using a turbular blender.

### Comparative Example 2

Comparative Example 2 is physically stable but chemically unstable. Table 2 shows the composition of Comparative Example 2. Table 3 shows the level of Benzophenone (Degradation product of diphenhydramine HCl) and the disintegration time of the tablets in comparison with Nytol tablets (comparative 3), after 6 months at 4°C and 40°C/75%RH.

**Table 2. Comparative Example 2**

| Ingredient | mg/tab | %w/w |
|---|---|---|
| Diphenhydramine HCL | 25 | 5.49 |
| Ibuprofen 50 | 200 | 43.96 |
| Croscarmellose sodium | 15 | 3.30 |
| Microcrystalline cellulose | 140 | 30.77 |
| Lactose BP | 70 | 15.38 |
| Calcium Stearate | 3 | 0.66 |
| Stearic Acid | 2 | 0.44 |
| Total | 455 mg | |

### Method of Manufacture

The Diphenhydramine HCl of comparative example 2 is blended with the croscarmellose sodium, microcrystalline cellulose and lactose. This mixture is then granulated using water. When the granule is dry the Ibuprofen, calcium stearate and stearic acid are added and the whole mixture is blended using a turbular blender.

**Table 3: Formulations Assayed at 6 months Time point**

| **Example** | **Ibuprofen 200mg/ Diphenhydramine HCl 25 mg tablets** | | **Level of Benzophenone** | | **Disintegration ratio time** |
|---|---|---|---|---|---|
| | **Ibuprofen Source** | **Diphen hydramine source** | % at 4°C | % at 40°C/ 75%RH | |
| Comparative 1 | Ibuprofen extrudate | Powdered Diphenhydramine HCl | 0.035 | 0.085 | Did not disintegrate rate |
| Comparative 2 | Powdered ibuprofen 50 | Diphenydramine granule (H₂O as granulating fluid) | 0.13 | 1.25 | < 2 min |
| Comparative 3 | N/A | Nytol tablets (Diphenhydramine HCl 50mg) | None | 0.04 | < 2 min |

Example 1 is both physically and chemically stable. Table 4 shows the composition of Example 1. Table 5 shows the level of Benzophenone (Degradation product of diphenhydramine HCl) and the disintegration time of the tablets in comparison with Nytol tablets (comparative 3), after 5 days at accelerated conditions of heat and humidity.

**Table 4. Example 1**

| Ingredient | mg/tab | %w/w |
|---|---|---|
| Diphenhydramine HCL | 25 | 5.52 |
| Microcrystalline cellulose | 100 | 22.08 |
| Lactose BP | 50 | 11.04 |
| Ibuprofen | 200 | 44.16 |
| Croscarmellose sodium | 30 | 6.62 |
| Sodium Citrate BP Pdr | 43.5 | 9.60 |
| Calcium Stearate | 2 | 0.44 |
| Stearic Acid | 2 | 0.44 |
| Total | 453 mg | |

### Method of Manufacture

The Diphenhydramine HCl is blended with the microcrystalline cellulose and Lactose. This mixture is then granulated using IPA. The dried granule is blended with milled extrudate (as in comparative example 1), Stearic acid and calcium Stearate.

**Table 5. Accelerated Stability Data (50°C) after 5 Days: Example 1 versus Comparative Example 3**

| **Example** | **Ibuprofen 200mg/ Diphenhydramine HCl 25 mg tablets** | | **Duration** | **Level of Benzophenone** | **Disintegration Time** |
|---|---|---|---|---|---|
| | Ibuprofen source | Diphenhydramine Source | | | |
| 1 | Ibuprofen extrudate | Diphenydramine granule (IPA as granulating fluid) | Initial | 0.01 | < 2 min |
| | | | 5 days | 0.06 | < 2 min |
| Comparative 3 | | Nytol tablets (Diphenhydramine HCl 50mg tablets) | Initial | None | < 2 min |
| | | | 5 days | 0.04 | < 2 min |

The jars were sealed and contained a vial of 1ml of water.

### *Extrusion Process

As mentioned above, the powder mixture of the ibuprofen is introduced into the heated chamber of an APV 40 mm diameter, twin screw extruder via a screw-feed hopper system. The ibuprofen alone melts, while the croscarmellose sodium, sodium lauryl sulphate and sodium citrate are insoluble and remain dispersed within the molten ibuprofen. The rate of feed is adjusted to ensure that the ibuprofen is satisfactorily molten when emerging from the extruder. Typical feed rates are in the range 120 - 180 kg per hour. The ingredients in the extruder are heated, typically within the temperature range 110°C to 130°C, and also worked by the screw arrangement in the extruder until the ibuprofen is fully molten. A continuous molten ribbon of extrudate is discharged from the extruder onto the surface of a cooled stainless steel belt. Diagrams of the extrusion system are shown in Figures 1-4. The extrudate cools and sets solid as it travels down the length of the moving cooled belt. At the end of the belt the solid mass is collected, milled using a cone mill to produce a suitable granulate and transferred for blending.

The invention is not restricted to the details of the foregoing embodiment(s).

## Claims

1. A tablet comprising at least a first and second active, the first active being in the form of a non-aqueous granulate, the second active ingredient being in the form of melt extrudated granules, the said first and second active forms having been blended together, optionally with other excipients, and formed into a tablet wherein the first active is an antihistamine selected from chlorpheniramine, brompheniramine, dexchlorpheniramine, dexbrompheniramine, triprolidine, diphenhydramine, doxylamine, tripelennamine, cyproheptadine, carbinoxamine, bromodiphenhydramine, phenindamine, pyrilamine, and azatadine, and wherein the second active is a suitable non-steroidal anti-inflammatory drug selected from the following categories:
a. the propionic acid derivatives; and
b. the acetic acid derivatives.

2. A tablet according to claim 1, wherein the first active is diphenhydramine or a pharmaceutically acceptable salt thereof.

3. A tablet according to claim 1 or claim 2, wherein a phenyl propionic acid is the NSAID and selected from ibuprofen, naproxen, ketoprofen and flurbiprofen.

4. A tablet according to any of claims 1 - 3, wherein the weight ratio of wet granulate:melt granules is in the range 1:10 to 5:1.

5. A tablet according to any of claims 1 - 4, wherein the melt granulate is ibuprofen and the ibuprofen:diphenhydramine weight ratio is between 50:1 to 1:2.

6. A method of producing a unit dosage form such as a tablet comprising the steps of:-
(i) producing a non-aqueous granulate of a first active wherein the first active is an antihistamine selected from chlorpheniramine, brompheniramine, dexchlorpheniramine, dexbrompheniramine, triprolidine, diphenhydramine, doxylamine, tripelennamine, cyproheptadine, carbinoxamine, bromodiphenhydramine, phenindamine, pyrilamine, and azatadine;
producing melt extruded granules of a second active and wherein the second active is a suitable non-steroidal anti-inflammatory drug selected from the following categories:
a. the propionic acid derivatives; and
b. the acetic acid derivatives
(ii) blending the said first and second active granules; and
(iii)forming the granules into a unit dosage form.

## Patentansprüche

1. Tablette, umfassend wenigstens einen ersten und einen zweiten Wirkstoff, wobei der erste Wirkstoff in der Form eines nichtwässrigen Granulats vorliegt, der zweite Wirkstoff in der Form von schmelzextrudierten Granulatkörnern vorliegt, die erste und die zweite Wirkstoffform zusammengemischt worden sind, gegebenenfalls mit anderen Hilfsstoffen, und zu einer Tablette geformt worden sind, wobei der erste Wirkstoff ein Antihistaminikum ausgewählt aus Chlorpheniramin, Brompheniramin, Dexchlorpheniramin, Dexbrompheniramin, Triprolidin, Diphenhydramin, Doxylamin, Tripelennamin, Cyproheptadin, Carbinoxamin, Bromdiphenhydramin, Phenindamin, Pyrilamin und Azatadin ist und wobei der zweite Wirkstoff ein geeignetes nichtsteroides entzündungshemmendes Arzneimittel ausgewählt aus den folgenden Kategorien ist:
a. den Propionsäurederivaten; und
b. den Essigsäurederivaten.

2. Tablette gemäß Anspruch 1, wobei der erste Wirkstoff Diphenhydramin oder ein pharmazeutisch verträgliches Salz davon ist.

3. Tablette gemäß Anspruch 1 oder Anspruch 2, wobei das NSAID eine Phenylpropionsäure ist und ausgewählt ist aus Ibuprofen, Naproxen, Ketoprofen und Flurbiprofen.

4. Tablette gemäß einem der Ansprüche 1-3, wobei das Gewichtsverhältnis von Nassggranulat:Schmelzgranulat in dem Bereich von 1:10 bis 5:1 liegt.

5. Tablette gemäß einem der Ansprüche 1-4, wobei das Schmelzgranulat Ibuprofen ist und das Ibuprofen:Diphenhydramin-Gewichtsverhältnis zwischen 50:1 und 1:2 beträgt.

6. Verfahren zur Herstellung einer Einheitsdosisform, wie z. B. einer Tablette, umfassend die Schritte:
(i) Herstellen eines nichtwässrigen Granulats eines ersten Wirkstoffs, wobei der erste Wirkstoff ein Antihistaminikum ausgewählt aus Chlorpheniramin, Brompheniramin, Dexchlorpheniramin, Dexbrompheniramin, Triprolidin, Diphenhydramin, Doxylamin, Tripelennamin, Cyproheptadin, Carbinoxamin, Bromdiphenhydramin, Phenindamin, Pyrilamin und Azatadin ist; Herstellen von schmelzextrudierten Granulatkörnern eines zweiten Wirkstoffs, wobei der zweite Wirkstoff ein geeignetes nichtsteroides entzündungshemmendes Arzneimittel ausgewählt aus den folgenden Kategorien ist:
a. den Propionsäurederivaten; und
b. den Essigsäurederivaten;
(ii) Mischen der ersten und der zweiten Wirkstoff-Granulatkörner; und
(iii) Formen der Granulatkörner zu einer Einheitsdosierungsform.

## Revendications

1. Comprimé comprenant au moins un premier principe actif et un deuxième principe actif, le premier principe actif se présentant sous la forme d'un granulat non aqueux, le deuxième principe actif se présentant sur la forme de granules extrudés à l'état fondu, lesdites formes du premier principe actif et du deuxième principe actif ayant été mélangées conjointement, éventuellement avec d'autres excipients, et façonnées en un comprimé, où le premier principe actif est un antihistaminique choisi parmi les suivants : chlorphéniramine, bromphéniramine, dexchlorphéniramine, dexbromphéniramine, triprolidine, diphénhydramine, doxylamine, tripélennamine, cyproheptadine, carbinoxamine, bromodiphénhydramine, phénindamine, pyrilamine et azatadine, et où le deuxième principe actif est un anti-inflammatoire non stéroïdien adapté choisi dans les catégories suivantes :
a. les dérivés d'acide propionique ; et
b. les dérivés d'acide acétique.

2. Comprimé selon la revendication 1, où le premier principe actif est la diphénhydramine ou l'un des sels pharmaceutiquement acceptables de celle-ci.

3. Comprimé selon la revendication 1 ou la revendication 2, où l'AINS est un acide phénylpropionique qui est choisi parmi les suivants : ibuprofène, naproxène, kétoprofène et flurbiprofène.

4. Comprimé selon l'une quelconque des revendications 1 à 3, où le rapport massique du granulat humide sur les granules fondus est compris dans l'intervalle allant de 1:10 à 5:1.

5. Comprimé selon l'une quelconque des revendications 1 à 4, où le granulat fondu est l'ibuprofène et le rapport massique de l'ibuprofène sur la diphénhydramine est compris entre 50:1 et 1:2.

6. Procédé de production d'une forme galénique unitaire telle qu'un comprimé comprenant les étapes consistant à :
(i) produire un granulat non aqueux d'un premier principe actif où le premier principe actif est un antihistaminique choisi parmi les suivants :
chlorphéniramine, bromphéniramine, dexchlorphéniramine, dexbromphéniramine, triprolidine, diphénhydramine, doxylamine, tripélennamine, cyproheptadine, carbinoxamine, bromodiphénhydramine, phénindamine, pyrilamine et azatadine ;
produire des granules extrudés à l'état fondu d'un deuxième principe actif, où le deuxième principe actif est un anti-inflammatoire non stéroïdien adapté choisi dans les catégories suivantes :
a. les dérivés d'acide propionique ; et
b. les dérivés d'acide acétique
(ii) mélanger lesdits granules du premier principe actif et du deuxième principe actif ; et
(iii) façonner les granules en une forme galénique unitaire.
